# EUROPEAN PATENT APPLICATION

(11) **EP 2 128 613 A1**
(43) Date of publication of application: **02.12.2009**
(21) Application number: 08157126.7
(22) Date of filing: 28.05.2008
(51) Int. Cl.: G01N 33/50, G01N 33/80

(54) **Method for screening compounds for their ability to increase rigidity of red blood cells infected by a protozoan parasite of the genus plasmodium and application thereof**

(71) Applicant: Institut Pasteur, 75724 Paris Cédex 15 (FR)
(72) Inventor: Buffet, Pierre Antoine Gérard, 75012 Paris (FR); Safeukui, Innocent, 75013 Paris (FR); Milon, Geneviève, 75012 Paris (FR); David, Peter, 75003 Paris (FR); Brousse, Valentine, 75014 Paris (FR); Puijalon, Odile, 92130 Issy Les Moulineaux (FR); Deplaine, Guillaume, 78280 Guyancourt (FR)
(74) Representative: Desaix, Anne

(57) **Abstract**

The invention relates to a method for screening compounds for their ability to increase rigidity of red blood cells (RBCs) infected by a protozoan parasite of the genus *Plasmodium* and in particular by *Plasmodium falciparum*. Said method comprises the following steps:
a) culturing RBCs infected by said parasite and, optionally and separately culturing uninfected RBCs, each culture being carried out both in the presence and in the absence of a compound to be tested for its ability to increase rigidity of infected RBCs (iRBCs) and;
b) measuring the deformability of one or several iRBCs cultured in the presence of said compound and of one or several iRBCs cultured in the absence of said compound; and,
c) optionally, measuring the deformability of one or several uninfected RBCs cultured in the presence of said compound, and one or several uninfected RBCs cultured in the absence of said compound,

wherein a decrease by at least 5%, preferably at least 10% and more preferably at least 15%, of the deformability of iRBCs cultured in the presence of a compound in comparison with the deformability of iRBCs cultured in the absence of the same compound is indicative that said compound is able to increase rigidity of iRBCs.

The invention also relates to the application of said method, for the selection of compounds which selectively interact with iRBCs or selectively interact with ring-iRBCs and are suitable to increase their rigidity.

## Description

The present invention relates to a method for screening compounds for their ability to increase rigidity of red blood cells infected by a protozoan parasite of the genus *Plasmodium* and in particular by *Plasmodium falciparum*.

The invention also relates to the application of said method, for the selection of compounds which selectively interact with red blood cells infected by a protozoan parasite of the genus *Plasmodium* and are suitable to increase their rigidity.

Protozoan parasites of the genus *Plasmodium* cause diseases (malaria) in humans and in many animal species. In humans, malaria is mainly caused by *Plasmodium falciparum, Plasmodium malariae, Plasmodium ovale, Plasmodium knowlesi* and *Plasmodium vivax. Plasmodium falciparum* is the most common cause of disease and is responsible for about 80% of all malaria cases, and is also responsible for about 90% of the deaths from malaria in humans. Parasitic *Plasmodium* species also infect animals including birds, reptiles and mammals, in particular monkeys, chimpanzees and rodents. Human infections with several simian species of *Plasmodium,* namely *Plasmodium knowlesi, Plasmodium inui, Plasmodium cynomolgi, Plasmodium simiovale, Plasmodium brazilianum, Plasmodium schwetzi* and *Plasmodium simium* have also been documented.

*Plasmodium falciparum* initially infects the liver, but then moves into the blood, where it multiplies and persists through an asexual replication cycle in red blood cells (also known as RBCs, haematids or erythrocytes). After invasion of a red blood cell, *Plasmodium falciparum* undergoes continuous phenotypic change and several rounds of mitotic division. After approximately 48 hours, the infected red blood cell bursts and releases free parasites, which either invade another red blood cell or are quickly (in less than half an hour) removed from the bloodstream by a variety of mechanisms.

The pathogenesis of malaria involves multiple parasite and host factors ¹. Spleen filtering and immune functions have a major impact on the course of plasmodial infection in experimental models ^{2,3}. In malaria-endemic countries, splenectomy predisposes to fever, to more frequent and higher parasitaemia (including circulating mature forms of the parasite), and may reactivate latent plasmodial infections ^{4,5}. Despite relatively few published data (reviewed in ⁵), clinicians include malaria in the list of infectious diseases justifying increased awareness in splenectomized non-immune patients ⁶. Because key features may differ between animal and human plasmodial infection, and because detailed exploration of the human spleen is limited by ethical and technical constraints ⁷, the fine interactions between *Plasmodium falciparum-infected* red blood cells (iRBCs) and the human spleen microcirculatory structures have been explored only indirectly ^{8,9} or post-mortem ¹⁰. Therefore, the mechanisms underlying the putative spleen protective or pathogenic effects during human malaria remain essentially speculative.

The architecture of the spleen red pulp (RP) permits intimate scrutiny of RBCs, leading to selective retention of abnormal or senescent RBCs within the RP ¹¹. To re-enter the venous system, RBCs leaving the reticular meshwork of the RP must cross the narrow inter-endothelial slits in walls of the venous sinuses. This process requires RBCs to undergo considerable deformation: if cells are not sufficiently deformable, they are retained upstream from the venous sinus wall¹¹. Such RBC-processing functions are expected to act upon RBCs hosting *Plasmodium*¹². Parasite development inside the RBC results in an altered host-cell membrane, which presents new structural, functional and antigenic properties, some of which are related to the peculiar severity of *Plasmodium falciparum* infections ^{13,14}. Ring-iRBCs are the predominant *Plasmodium falciparum* forms found in the circulation, in contrast with tissue sequestration of cytoadhering mature-iRBCs. Deformability of mature-iRBC is markedly reduced, which would result in the retention in the spleen of those escaping sequestration in "classical" vascular beds ¹⁵. In this context, a quantified spleen circulatory framework is an essential prerequisite for a consistent understanding of malaria pathogenesis. Previous experimental determination of the proportion of spleen blood flow directed to the filtering beds of the open circulation in animals ranged from 90% ¹⁶ to 10% ¹¹, warranting direct *in vivo* explorations in humans.

We report here on complementary dimensions of human spleen physiology and malaria pathogenesis, following two conceptually connected approaches: *in vivo* imaging in healthy volunteers, and challenge of an *ex vivo* human spleen perfusion system ⁷ with cultured iRBCs. We provide here the first *in vivo* confirmation of a two-compartment blood circulation in the human spleen, the slower compartment accounting for 10% of the flow. We show that, at each spleen passage, 10% of a previously ignored sub-population of ring-iRBCs is retained in the slow, open circulatory structures of the human spleen RP, most probably by a mechanical process. These results uncover heterogeneity of ring stage parasites and provide a new vision of RBC filtration by the spleen, shedding new light on control of parasite multiplication and RBC loss in malaria patients.

### BRIEF DESCRIPTION OF THE FIGURES

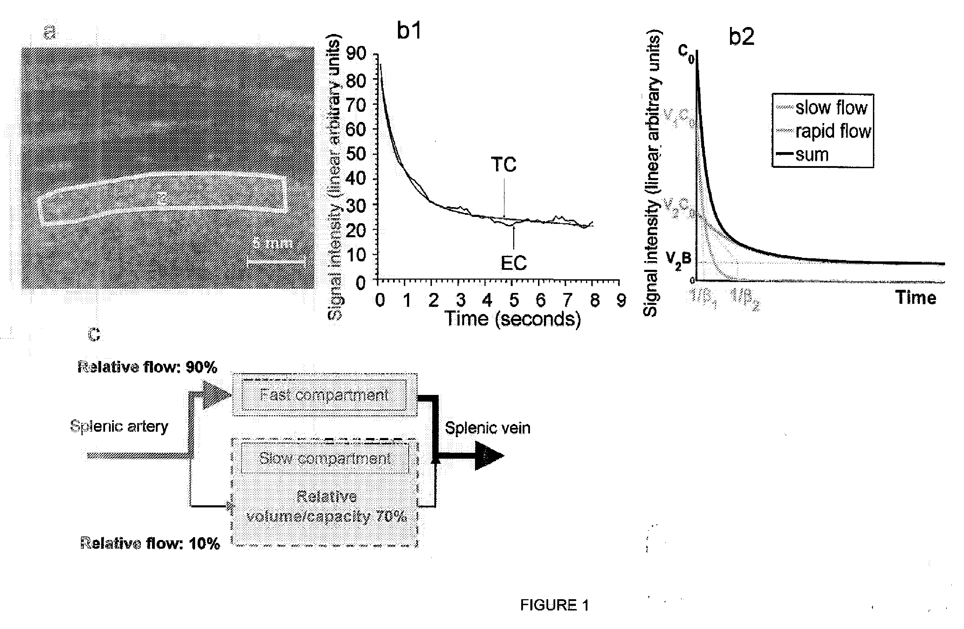

**Figure 1****: Contrast ultrasound analysis of blood circulation in the human spleen parenchyma. (a)** Enhancement of the ultrasound signal intensity in the spleen of a human volunteer receiving a constant perfusion of Sonovue microbubbles. **(b1)** The ultrasound-induced decrease of signal intensity ⁴³ in a sub-capsular zone [white line in (a)] was studied for ≥ 8 seconds. The best fit to the resulting Experimental signal-time Curve (EC) was obtained with a bi-exponential curve (TC), as shown on this typical example (the correlation coefficient R2 was > 0.96 in all 16 volunteers). **(b2)** Graphic representation of the bi-exponential mathematical model of signal intensity versus time N(t) = V₁(C₀exp(-β₁t)) + V₂(B + (C₀-B)exp(-β₂t)), where the first and second terms correspond to a slow, and a rapid flow compartment, respectively. (c) Schematic representation of the blood circulation in the human spleen parenchyma derived from these observations.

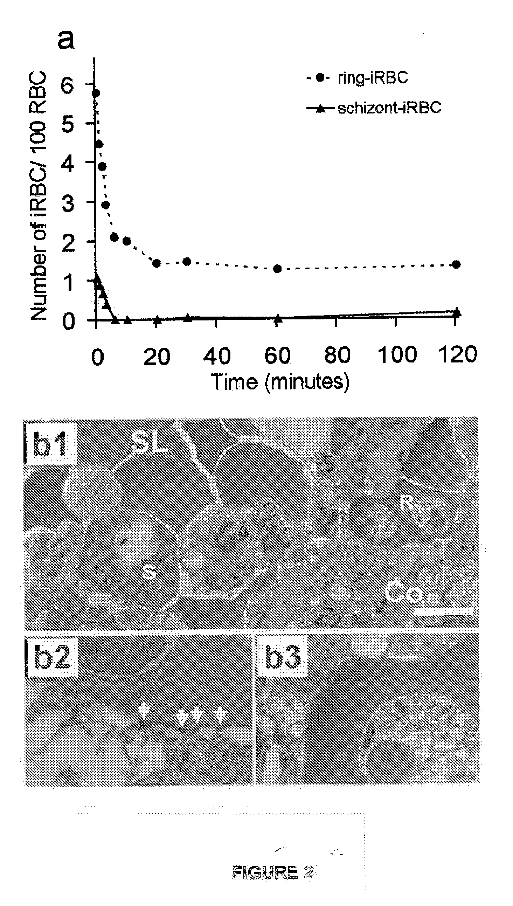

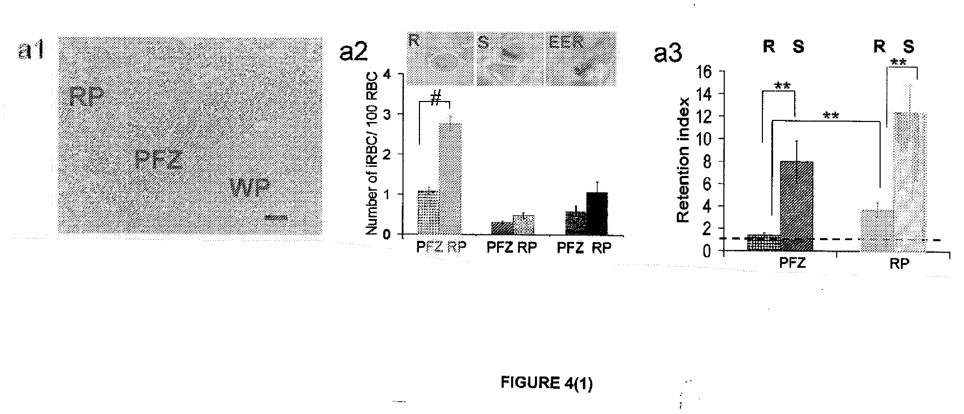

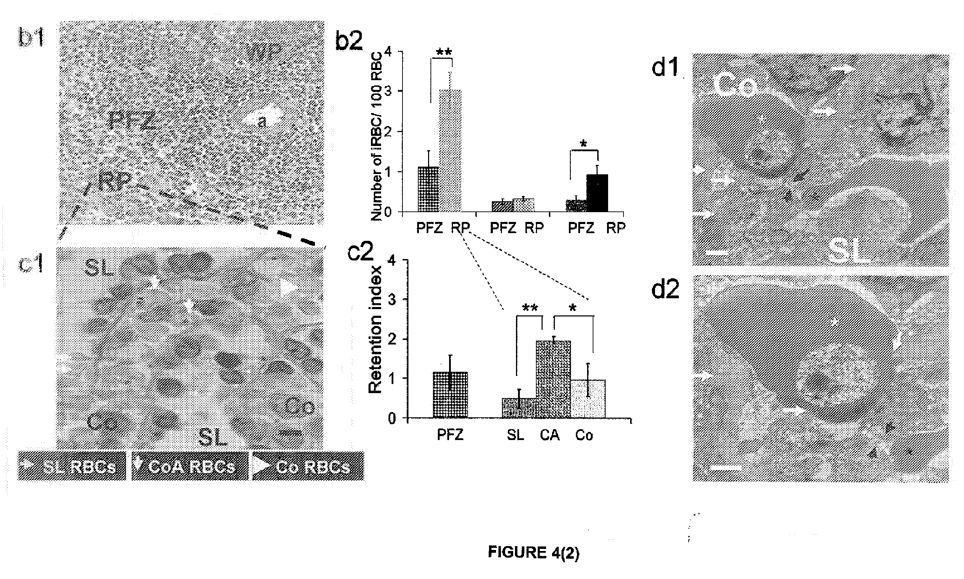

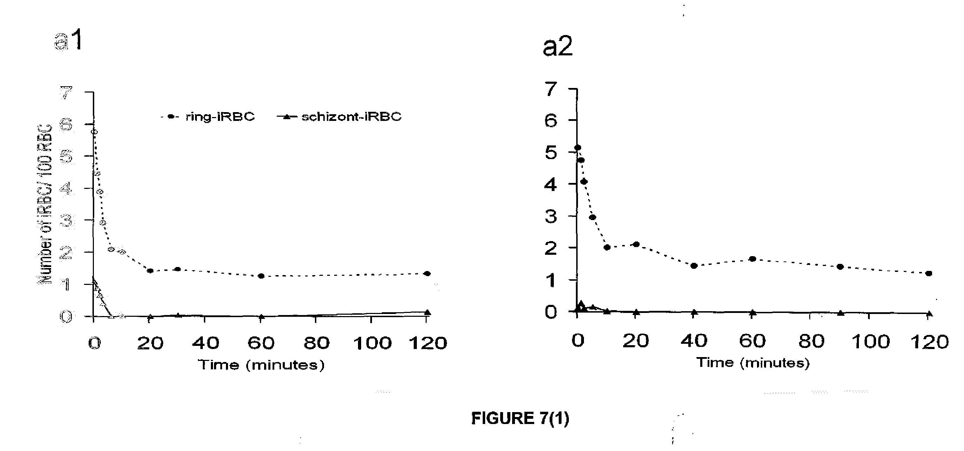

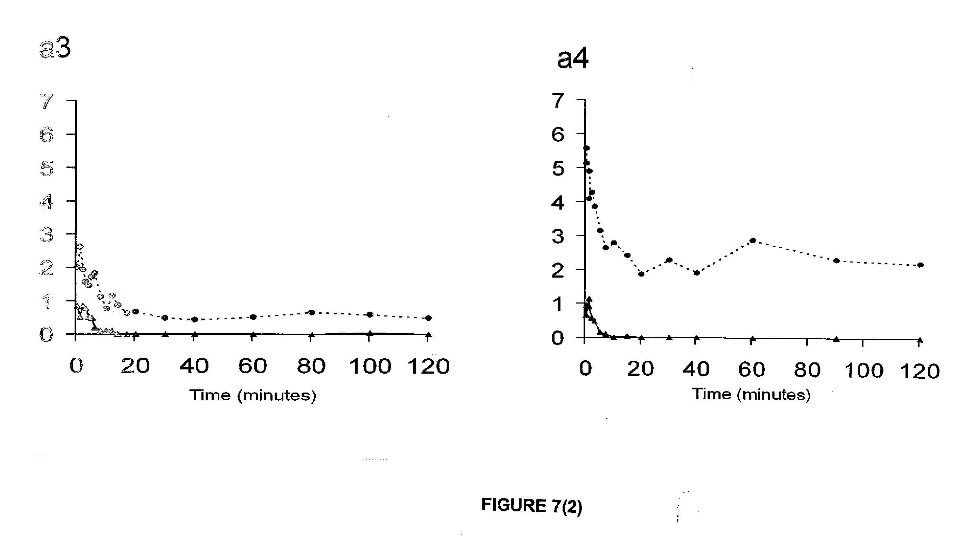

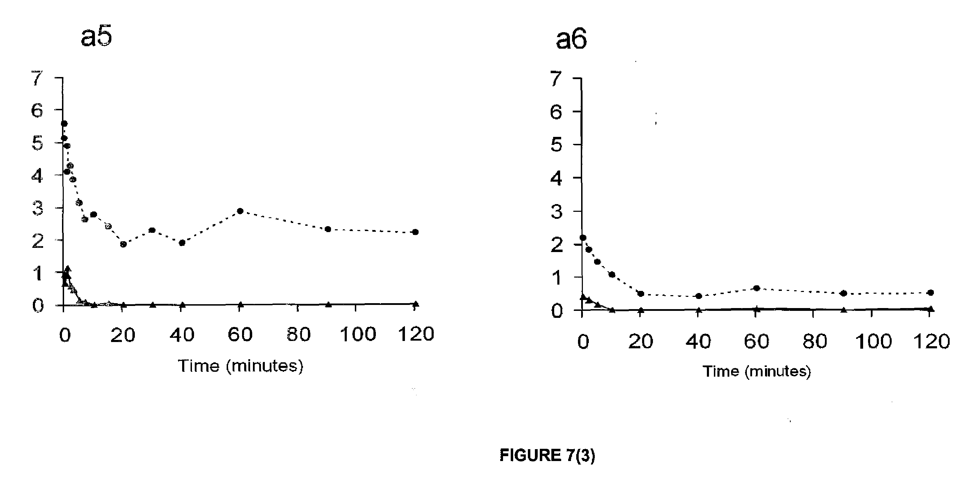

**Figure 2****: Clearance of iRBCs by the isolated-perfused human spleen. (a)** Parasitaemia of *Plasmodium falciparum* FUP schizont-iRBCs (Δ), and ring- iRBCs (•) in the perfusate during 120 minutes of perfusion in 1 of 6 independent experiments with isolated-perfused human spleens (all 6 experiments shown in figure 7 a1-6). **(b)** Transmission electron microscopy of an end-experiment spleen sample showing a schizont-iRBC [S, b1-2, (bar = 1 with knobs (b2, arrows) in the sinus lumen (SL, b1) of the red pulp, and a knobless ring iRBC (R, b1-3), in the cords (Co, b1). Another example of retained ring-iRBC is shown in Figure 4 (d1-2).

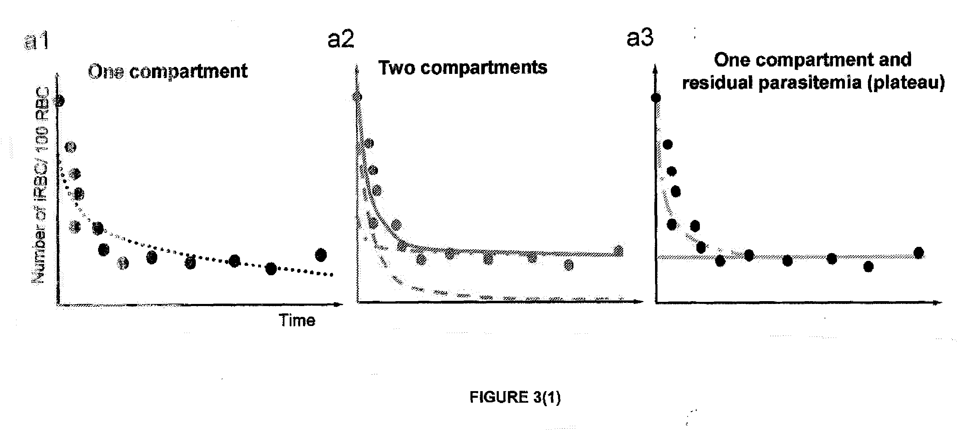

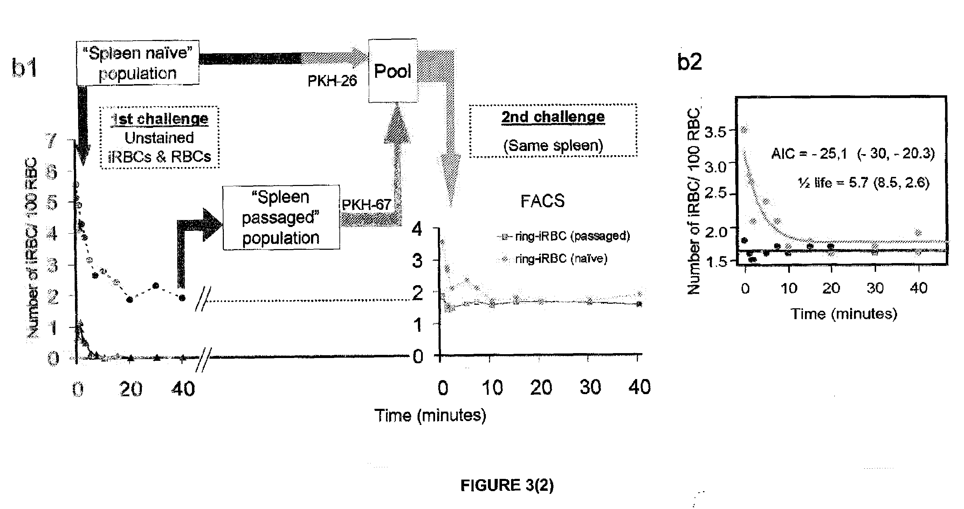

**Figure 3****: Modeling of ring-iRBC clearance during "circulation" and "circulation-recirculation" experiments. The uncovering of two ring-iRBC sub-populations. (a)** Modeling of ring-iRBC parasitaemia in the perfusate, graphically illustrated by a theoretical example: one-compartment without a residual parasitaemia **(a1),** two compartments without a residual parasitaemia (**a2**, the plain line corresponds to the bi-exponential curve, the dotted lines to the mono-exponential curves), and one-compartment with a residual parasitaemia (plateau phase, a3). The evaluation of the goodness of fit and the estimated parameters was based on the Akaike Information Criterion (AIC), the variability (VC) of the parameter estimates (the lower the AIC & VC values, the more parsimonious the model) and the random distribution of weighted residuals between measured and predicted concentrations with respect to time. Individual values are shown in Table 1. The AIC was lower for the two-compartment model and the 1-compartment model with residual parasitaemia than for the one-compartment model without a residual parasitaemia. However, for a two-compartment model the coefficient of variation of the second half-life parameter was not significant (> 100%). So, the one-compartment model with residual parasitaemia was the best model. **(b)** "Circulation - recirculation" experiments. Part of the iRBC and RBC population prepared for a spleen challenge ("Spleen naïve" population) was kept at 37°C in perfusion medium while the other part was perfused through the spleen. **(b1).** Forty minutes post the perfusion onset, iRBCs and RBCs were retrieved from the circulating perfusate ("Spleen passaged" population, b1). Both populations were differentially labeled using either PKH-26 or PKH-76, then pooled and reintroduced into the perfusate, 110-120 minutes post the initial perfusion step. Clearance kinetics of each iRBC subpopulation established using flow-cytometry showing that spleen "naïve" ring-iRBCs were cleared, whereas iRBCs previously submitted to ≈ 40 spleen passages were not. The most parsimonious model of the kinetics of the "spleen naive" population was again one-compartment with a residual parasitaemia. Mean (individual values) AIC and half-life from two independent experiments were similar to that observed during the 6 previous "Circulation" experiments **(b2).** The most parsimonious model for the "spleen passaged" population was a residual parasitaemia without elimination (see Table 1).

**Figure 4****: Analysis of *Plasmodium falciparum*-iRBC deposition in the human spleen. (a1-2)** Mean (SEM) number of iRBCs/100RBC in the peri-follicular zone (PFZ) or in the red pulp (RP) for ring-iRBCs (R, in PFZ, in RP), schizont-iRBCs (S, in PFZ, in RP), or Extra-Erythrocytic parasite Remnants (EER, in PFZ, in RP) subpopulations (mean from 6 isolated perfused human spleen experiments, Giemsa-stained sections, WP= White Pulp, bar = 50 _m). Typical aspects of each parasite development stage are shown on the inserts (middle column). **(a3)** Retention index [RI = ("number of iRBC/100RBC" as in a2)/(circulating parasitaemia at the end of the corresponding experiment)] for ring-iRBCs (R) or schizont-iRBCs (S) either in the PFZ or in the RP. A Retention Index of 1 (black interrupted line) corresponds to absence of retention. **(b1-2)** Same approach as in a1-2, but using PAS-staining (a = Central artery). **(c1)** Purple PAS-stained section (bar = 5µm) showing the basal membrane of red pulp venous sinuses. Working classification of RBC deposition in the spleen red pulp: in the sinus lumen (SL), in the cords in direct contact with the sinus wall basal membrane (cordal abluminal, CoA), or in the cords but without contact with the basal membrane (cordal *stricto sensu*, Co). **(c2)** Retention index for ring-iRBCs using the working classification of RBC deposition in the RP defined in c1. **(d1-d2)** Transmission electron microscopy of a human isolated-perfused spleen sample showing a ring-iRBCs (white star) and an uninfected RBC (black star) upstream and downstream of an inter-endothelial slit (black arrows), respectively. This disposition reflects the cord-(Co) to-sinus lumen (SL) circulation of RBC in the spleen red pulp. The knobless membrane of the ring-iRBC is very close to the sinus wall basal membrane (white arrows). For panels a, b & c: *, **, # mean statistically significant difference with p < 0.05, p ≤ 0.01 et # p < 0.0001, respectively.

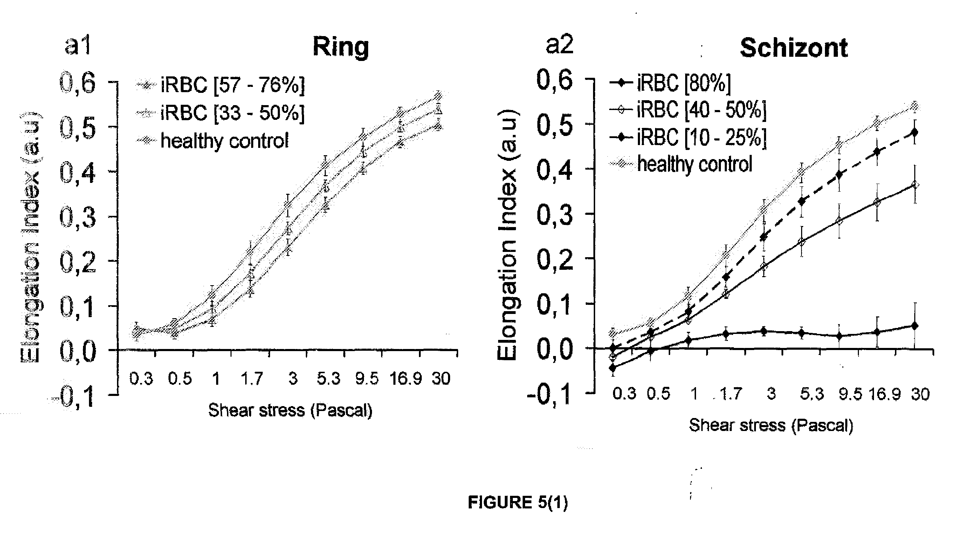

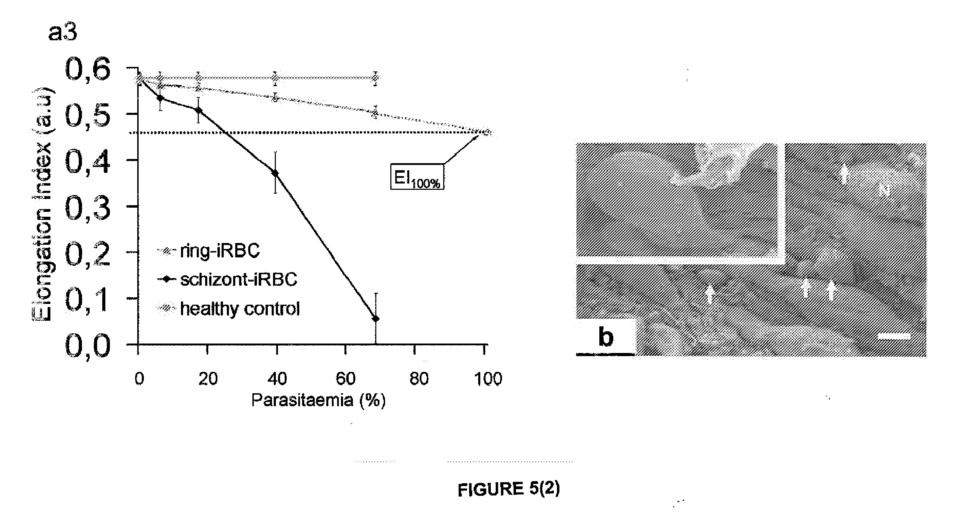

**Figure 5****: Deformability of iRBCs. (a)** Elongation index (EI) of red blood cells measured at different shear stresses **(a1-2). (a3)** EI of cultured ring-iRBC and schizont-iRBC at increasing parasitaemia at 30 Pascal (mean and standard deviation (SD) of 4 independent experiments). The mean [95% Confidence Interval (Cl)] of the extrapolated El of ring-iRBC at 100% parasitaemia is 0.47 [0.43 - 0,51]. **(b)** Scanning electron microscopy of spleen samples processed at the end of the perfusion. Endoluminal view of sinus walls showing the typical string-like aspect of endothelial cells, with their nuclei (N) protruding in the sinus lumen. Cells are emerging from inter-endothelial slits (arrows, bar = 3 µm). Upper left quadrangle: marked deformation of RBCs squeezing through inter-endothelial slits.

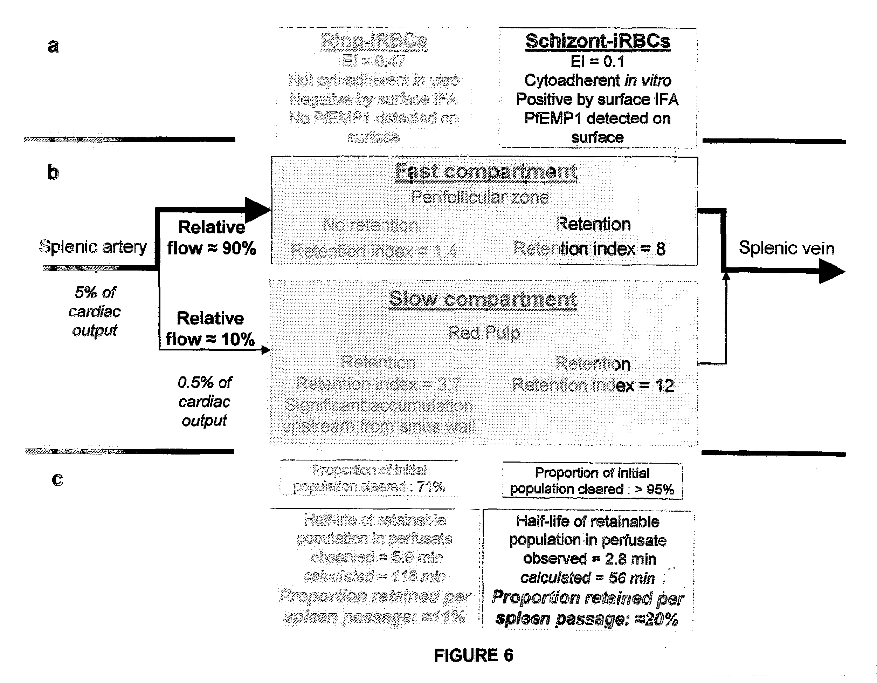

**Figure 6****: *Plasmodium faiciparum*-infected RBCs through the fast and slow circulatory compartments of the perfused human spleen.** Schematic representation of observations in the *ex-vivo* human spleen model challenged with cultured iRBCs (see Figures 2-5), using the circulatory features extracted from *in vivo* imaging in human volunteers as a framework (Figure 1). Ring- and schizont-iRBCs differed in their main phenotypic characteristics, ring-iRBC displaying no cytoadherent properties *in vitro* and no observable surface modifications. In the fast compartment, corresponding to the perifollicular zone on histological sections, only schizont-iRBC were retained, whereas both ring-and schizont-iRBcs were retained in the slow compartment, corresponding to the red pulp (b). This resulted in significantly different clearance rates. The similarity between the proportion of blood flowing to the slow compartment (10.2%) and the proportion of retainable ring-iRBCs retained in the same compartment (11%) was striking. The quantitative dimension of the spleen microcirculatory framework is important. Because, 5% of the cardiac output goes to the spleen, a given RBC will enter the spleen every 20 minutes. The slow compartment accounts for only 10% of the spleen plasma flow, which corresponds to 10 - 20% of spleen red blood cell flow (depending on the intensity of plasma skimming effect ¹¹). Therefore, the quality control of the deformability of a given RBC occurs every 100-200 minutes. This fits with the 60-minute half-life of stiff heated RBCs previously observed in healthy controls ³⁹. The order of magnitude of those previous clinical observations perfectly fits our framework.

**FIGURE 7****: (a1-6)** Parasitaemia of *Plasmodium falciparum* FUP schizont-iRBCs (Δ triangles), and ring-iRBCs (• circles) in the perfusate during 120 minutes of perfusion (6 independent experiments).

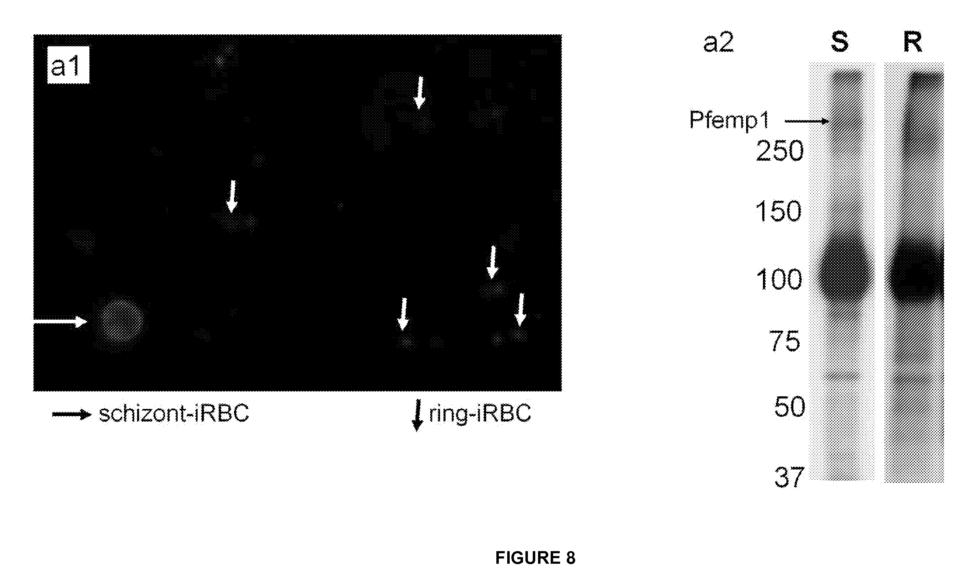

**FIGURE 8**. (a) Surface immunofluorescence using a pool of hyperimmune sera from adult African patients (1/100 dilution) of cultured schizont-iRBC (a1) and ring-iRBC (a2) populations prepared for experiments, and counter-stained with Hoechst. The typical Giemsa-stained aspect is shown on inserts. Most schizont-iRBCs were intensely stained whereas no ring-iRBC was stained (bar = 10 µm). (a3) PAGE auto-radiographs of Triton-SDS extracts from surface-iodinated cultured schizont-iRBCs (S), and ring-iRBCs (R). The 290 kDa band (arrow) probably corresponding to PfEMP1, was observed only in the schizont-iRBC extract.

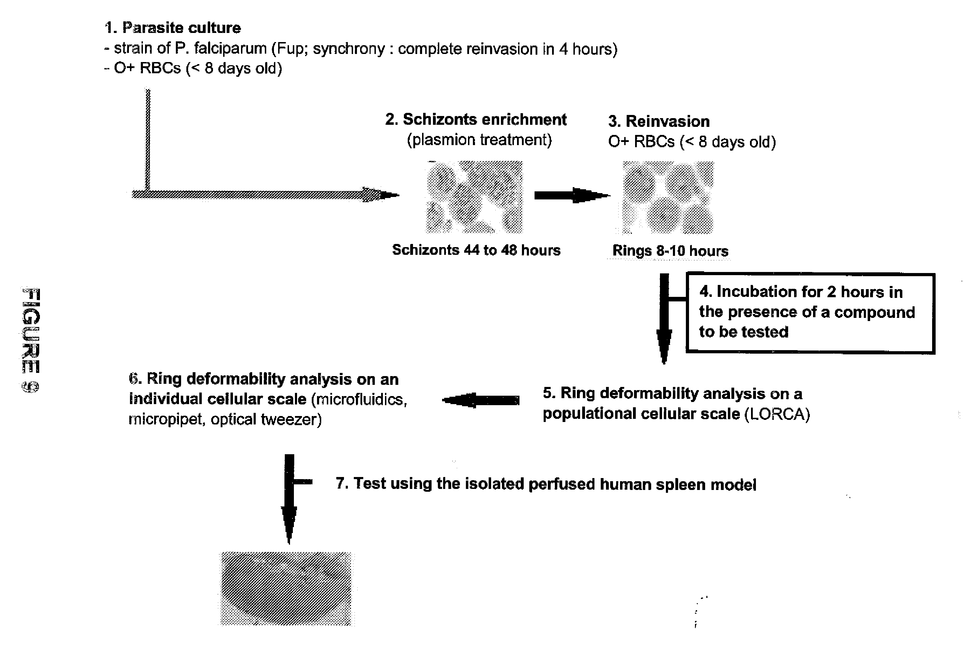

**FIGURE 9****.** Schematic representation of experimental steps that can be carried out to screen for compounds increasing rigidity of *Plasmodium falciparum* ring-infected RBCs.

### DETAILED DESCRIPTION OF THE INVENTION

The present invention relates to a method for screening compounds for their ability to increase rigidity of red blood cells (RBCs) infected by a protozoan parasite of the genus *Plasmodium*, said method comprising or consisting of the following steps:
a) culturing RBCs infected by said parasite and optionally and separately culturing uninfected RBCs, each culture being carried out both in the presence and in the absence of a compound to be tested for its ability to increase rigidity of iRBCs and;
b) measuring the deformability of one or several iRBCs cultured in the presence of said compound, and one or several iRBCs cultured in the absence of said compound; and,
c) optionnaly, measuring the deformability of one or several uninfected RBCs cultured in the presence of said compound, and one or several uninfected RBCs cultured in the absence of said compound,
   wherein a decrease by at least 5%, preferably at least 10% and more preferably at least 15%, of the deformability of iRBCs cultured in the presence of a compound in comparison with the deformability of iRBCs cultured in the absence of the same compound is indicative that said compound is able to increase rigidity of iRBCs.

In a particular embodiment, said method comprises or consists of the following steps:
a) culturing RBCs infected by said parasite and separately culturing uninfected RBCs, each culture being carried out both in the presence and in the absence of a compound to be tested for its ability to increase rigidity of iRBCs and;
b) measuring the deformability of:
   - one or several iRBCs cultured in the presence of said compound,
   - one or several iRBCs cultured in the absence of said compound,
   - one or several uninfected RBCs cultured in the presence of said compound, and
   - one or several uninfected RBCs cultured in the absence of said compound,
      wherein a decrease by at least 5%, preferably at least 10% and more preferably at least 15%, of the deformability of iRBCs cultured in the presence of a compound in comparison with the deformability of iRBCs cultured in the absence of the same compound is indicative that said compound is able to increase rigidity of iRBCs.

The "compound" as used herein can be any chemical compound, immunoglobulin (Ig), polypeptide, peptide, or other biotherapeutics that is able to increase rigidity of iRBCs. Chemical agents, referred to in the art as "small molecule" compounds are typically organic, non- peptide molecules, having a molecular weight up to 10,000, preferably up to 5,000, more preferably up to 1,000, and most preferably up to 500 Daltons. Ig can be generated using known methods and may be for example polyclonal, monoclonal, humanized or chimeric antibodies, single chain antibodies or Fab fragments. A "peptide" or "polypeptide" is any chain of two or more amino acids (2-20 amino acids for a "peptide" and more than 20 amino acids for a "polypeptide"), including naturally occurring or non-naturally occurring amino acid residues and amino acid residue analogues, regardless of post-translational modification (e.g., glycosylation or phosphorylation). Such a compound may be obtainable from or produced by any suitable source, whether natural or not. Where appropriate, said compound may be synthesized by any chemical or biological synthesis techniques.

According to a particular embodiment, a bank of compounds is screened. In particular, the bank from Sanofi Aventis can be screened.

According to a particular embodiment, the screened compounds are capable of interacting with the RBCs membrane its cortical cytoskeleton included (the iRBCs membrane its cortical cytoskeleton included at least) and/or entering into the RBCs (into the iRBCs at least), in particular crossing the RBCs lipid bilayer (the iRBCs lipid bilayer at least), or interact with the modified bilayer itself.

According to a particular embodiment, the screened compounds selectively interact with iRBCs by increasing their rigidity. By "selectively interact with iRBCs" it is meant herein that the selected compounds interact with iRBCs and increase their rigidity whereas they do not increase the rigidity of uninfected RBCs and preferably of other types of cells. This means that one screens the deformability of uninfected RBCs cultured in the presence of the compound which is approximately the same or differs by less than 5% from that of the uninfected RBCs cultured in the absence of the compound.

According to a particular embodiment, the screened compounds selectively interact with ring-iRBCs by increasing their rigidity. By "selectively interact with ring-iRBCs" it is meant herein that the selected compounds interact with ring-iRBCs and increase their rigidity whereas they do not increase the rigidity of iRBCs at the schizont stage (schizont-iRBCs), nor of uninfected RBCs nor preferably of other types of cells. This means that one screens (i) the deformability of schizont-iRBCs cultured in the presence of the compound which is approximately the same or differs by less than 5% from that of the schizont-iRBCs cultured in the absence of the compound, and (ii) the deformability of uninfected RBCs or of other types of cells cultured in the presence of the compound which is approximately the same or differs by less than 5% from that of the uninfected RBCs or of other types of cells cultured in the absence of the compound.

By "ability to increase rigidity of iRBCs", it is meant herein the ability to increase rigidity of iRBCs by at least 5%, preferably at least 10% and more preferably at least 15%. The ability of a compound to increase rigidity of iRBCs can be in particular assessed by measuring the deformability of iRBCs cultured in the presence and in the absence of said compound. Thus, according to a particular embodiment, "increase rigidity" means "decrease deformability" and in particular "decrease deformability by at least 5%, preferably at least 10% and more preferably at least 15%".

By "infected by a protozoan parasite of the genus *Plasmodium*", it is meant herein red blood cells that contain at least one parasite, (i.e., a daughter cell of said protozoan parasite, which is the result of asexual reproduction), of the genus *Plasmodium.*

According to one embodiment, the parasite is chosen in the group consisting of *Plasmodium falciparum, Plasmodium vivax, Plasmodium ovale, Plasmodium malariae, Plasmodium knowlesi, Plasmodium inui, Plasmodium cynomolgi, Plasmodium simiovale, Plasmodium brazilianum, Plasmodium schwetzi* and *Plasmodium simium*, preferably in the group consisting of *Plasmodium falciparum, Plasmodium vivax, Plasmodium ovale, Plasmodium knowlesi* and *Plasmodium malariae,* and more preferably in the group consisting of *Plasmodium falciparum* and *Plasmodium vivax*.

In a particular embodiment, said parasite is *Plasmodium falciparum,* in particular the Palo Alto I strain of *Plasmodium falciparum*.

According to a particular embodiment, the parasite(s), in particular the merozoite(s) that have infected RBCs have developed into ring forms; hence, said infected RBCs are at the ring stage. In particular, fresh ring-infected RBCs (ring-iRBCs), i.e. ring-iRBCs of less than 14 hours, in RBCs of less than 8 days or of 8 days of age (preferably RBCs of less than 8 days of age), can be collected.

Ring-iRBCs can be selected by artificially imposing synchrony upon developing *Plasmodium* parasites. Several methods are known in the art, for example the use of sorbitol or mannitol treatment; treatment of iRBCs with 5% sorbitol (for example 5 minutes at 37°C) causes lysis of iRBCs containing late stages and preferentially selects for iRBCs with early ring stages. Treatment can be repeated (at least two times, for example, 2, 3, 4 or 5 times or more than 5 times), for example after 34 hours, to further select young stages and improve on the synchrony. Other techniques involve the separation of late-stage parasites, for example sedimentation in Plasmagel or gelatin. Thus, in one embodiment, step a) of the method of the invention is preceded by a step of synchronization of the parasites of the iRBC culture.

According to one embodiment, RBCs (uninfected and/or infected RBCs) are human RBCs. RBCs from simian primates could also be used but human RBCs are more preferably used. Human RBCs of all blood groups are suitable for *plasmodium* growth, and are particularly suitable for *Plasmodium falciparum* growth. Type O RBCs are especially useful because of their compatibility with serum or plasma of all other blood groups.

The screening performed can be a low throughput screening or a high throughput screening. Alternatively, the method of the invention can comprises one or several step(s) of low throughput screening and one or several step(s) of high throughput screening.

The measure of the deformability of RBCs and iRBCs is a phenotypical measure. It can be performed on a populational cellular scale and/or on an individual cellular scale. Thus, the method of the invention comprises one or several step(s) of deformability analysis on a populational cellular scale and/or one or several step(s) of deformability analysis an individual cellular scale.

In order to enable assessment of the deformability of iRBCs, and in particular of ring-iRBCs, RBCs and iRBCs are generally cultured at 37°C, in RPMI 1640 medium or supplemented RPMI 1640 medium, such as complete medium (RPMI 1640 medium, bicarbonate, 25mM glutamine, 0.2% glucose, 100 µM hypoxanthine, 10 µg/ml gentamicine and 10% AB+ inactivated human serum pool). In addition, these cells are preferably cultured in low oxygen pressure (low p0₂) conditions, for example, in an atmosphere of 1% O₂, 3% CO₂ and 96% N₂.

RBCs and iRBCs can be incubated from 0.1 to 10 hours, preferably from 1 to 6 hours, and more preferably from 1 to 3 hours, for example 1, 2 or 3 hour(s) with the compound to be tested. Thus, in the method of the invention, step b) can be performed from 0.1 to 10 hours, preferably from 1 to 6 hours, and more preferably from 1 to 3 hours, for example 1, 2 or 3 hour(s), after step a).

Deformability analysis is generally performed after (i) removal of the culture supernatant (for example by centrifugation at 1500 rounds per minute for 5 minutes) and (ii) resuspension of the pellet in PBS.

Various techniques in measuring the deformability of RBCs have been proposed, in particular ektacytometry, rheoscopy, microfluidics, micropipet aspiration and the use of optical tweezers. Thus, according to one embodiment, the deformability of RBCs and iRBCs is measured by means of a rheoscope, an ektacytometer, a microfluidic device (in particular a capillary microfluidic system), a micropipet and/or an optical tweezer.

Ektacytometry uses laser diffraction analysis of RBCs under varying shear stress levels. This technique has several advantages compared to other techniques, in that it is relatively easy to perform, has acceptable precision, can be performed at various shear stresses, and enables to measure rapidly cellular deformability using extremely small quantities of blood (less than 50µl). Automated ektacytometers are commercially available. Models that can be used in the method of the invention include LORCA (Laser-assisted Optical Rotational Cell Analyzer; Mechatronics, Hoorn, Netherlands) and RHEODYN-SSD (Myrenne, Roetgen, Germany). Ektacytometry provides a measure of cell deformability by determining the elongation index values of the cells under fluid shear stress using laser diffractometry and image analysis. RCBs are exposed to increasing shear stress and the laser diffraction pattern through the suspension is recorded. The laser diffraction pattern goes from circular to elliptical as shear stress value increases. From these measurements a deformability index for the cells can be derived.

According to a particular embodiment, the ektacytometer used to measure deformability of RBCs and iRBCs is the commercial rotating type ektacytometer, LORCA. Using such an ektacytometer, a viscous suspension of RBCs is sheared between two concentric cylinders. As indicated in the example part, the rotation of one of the cylinder causes deformation (elongation) of the RBCs. The laser beam diffraction pattern is detected with a video camera and analyzed by a computer which converts it into a digital value, the elongation index (EI).

Thus, in one embodiment, and in particular when an ectacytometer is used, the measure of deformability is carried out by reference to the elongation index (EI). This measure, which is generally defined as the ratio between the difference between the two axes of the ellipsoid diffraction pattern and the sum of these two axes, is usually determined from an intensity curve in the diffraction pattern using an ellipse-fitting program. Techniques for measuring RBC deformability often result in an indication of the mean value when deformability is analysed on a populational cellular scale.

In addition, in one embodiment and especially when an ectacytometer is used, deformability of RBCs and iRBCs, and in particular the elongation index, is usually measured over a range of shear stresses from 0 to 35 Pascal (Pa), preferably from 0.3 to 30 Pa, for example from 1.7 Pa to 30 Pa, and in particular at 1.7 Pa and/or at 30 Pa.

According to a particular embodiment, the parasitemia level of iRBCs is of at least 20% or 30% of the cell population, preferably at least 50% and more preferably at least 70% of the cell population.

By "parasitemia", it is meant the quantitative content of parasites expressed in the percentage of RBCs containing at least one ring in the culture. The number of plasmodia can be counted, for instance, using an optical microscope, (for low parasitemias) or thin film blood smear (for high parasitemias).

In general, and especially when deformability of RBCs and iRBCs is measured on a populational cellular scale (for example when an ektacytometer is used), the EI is measured at different parasitaemia (at least at two different parasitaemia), for example from 4% to 76% parasitaemia for rings-iRBCs, and extrapolated to 100% parasitaemia.

According to one embodiment, an EI below 0.43, more preferably below 0.42, for iRBCs cultured in the presence of the compound to be tested, when extrapolated at 100% parasitemia, at 30 Pa, is indicative that said compound is able to increase rigidity of iRBC.

Parasitemia can be induced or increased in a culture of RBCs (uninfected or already infected RBCs) by infecting (or re-infecting) said RBCs with a culture of iRBCs, in particular with a culture containing iRBCs at the schizont stage. iRBCs at the schizont stage can be enriched in a culture of iRBC for example by suspension in a gelatinous solution (plasmion treatment) or by any other technique known in the art, in particular using a percoll gradient.

According to a particular embodiment, the iRBCs population used in step a) of the method of the invention has been obtained by infecting uninfected RBCs, in particular fresh uninfected RBCs (i.e., RBCs of less than 8 days or of 8 days of age, preferably RBCs of less than 8 days of age) with a culture of iRBCs, and in particular with a culture in which the schizont-stage iRBCs have been enriched, for example by plasmion treatment. In a particular embodiment, deformability is thus measured using iRBCs which have only been submitted to culture and not to any other treatment.

Hence, according to a particular embodiment, the method of the invention comprises or consists of the following steps:
1) enriching the schizont-stage iRBCs in a culture of iRBCs, for example by plasmion treatment or by any other technique known in the art;
2) infecting RBCs, in particular fresh RBCs (i.e., RBCs of less than 8 days or of 8 days of age, preferably RBCs of less than 8 days of age) with the schizont-enriched iRBCs culture of step 1) (this second step allows to obtain iRBCs at the ring stage);
3) culturing the iRBCs obtained at step 2) and, optionally and separately culturing uninfected RBCs, each culture being carried out both in the presence and in the absence of a compound to be tested for its ability to increase rigidity of iRBCs and;
4) measuring the deformability of one or several iRBCs cultured in the presence of said compound and one or several iRBCs cultured in the absence of said compound; and,
5) optionally, measuring the deformability of one or several uninfected RBCs cultured in the presence of said compound and one or several uninfected RBCs cultured in the absence of said compound,
   wherein a decrease by at least 5%, preferably at least 10% and more preferably at least 15%, of the deformability of iRBCs cultured in the presence of a compound in comparison with the deformability of iRBCs cultured in the absence of the same compound is indicative that said compound is able to increase rigidity of iRBCs.

An example of such a method of screening is given in the example part (see example B).

In addition, in the above-mentioned particular embodiment, the parasites of the iRBCs cultures, in particular the parasites of the schizont-enriched iRBCs culture can be synchronized by any method known in the art, in particular by any synchronization method cited herein, for example by one or several (2, 3, 4 or 5 or more than 5) sorbitol treatment(s). Said synchronization step or at least one synchronization step is preferably performed before the step of schizont- enrichment.

According to one embodiment, the method of the invention further comprises a step of validating a screened compound with an isolated perfused human spleen model and/or an isolated perfused pig spleen model. Said further step is generally performed after the step(s) of deformability analysis.

The present invention also relates to the application of the method of the invention, for the selection of compounds which selectively interact with iRBCs or selectively interact with ring-iRBCs and are suitable to increase their rig id ity.

### EXAMPLES

### A. Retention of Plasmodium falciparum ring-infected erythrocytes in the slow, open micro-circulation of the human spleen

### PATIENTS, MATERIAL AND METHODS

**Contrast-enhanced ultrasonography in human volunteers.** Sixteen 18 - 50 year-old healthy male volunteers were enrolled according to the following inclusion criteria : no risk factor for atheroma (i.e, normal arterial pressure, no present or past smoking habit, normal serum cholesterol level), no previous history of abdominal surgery, no present or past significant health problem (normal physical examination, normal serum creatinine, AST and bilirubin levels, and absence of antibody to HIV, HBV and HCV), normal splenic macrovascular structures as assessed by conventional ultrasonography and Doppler, and no clinical or biological indication of red blood cell disease - including normal blood cell count, serum haptoglobin, and hemoglobin electrophoresis. The study was approved by the Necker Hospital Investigational Review Board, and written informed consent was obtained from all participants. An ultrasound contrast agent (Sonovue°, Bracco, Milano, Italy) was injected intravenously at a constant infusion rate of 1ml/min. Contrast-enhanced ultrasonography was performed using a Philips HDI 5000 (Philips US, Bothell, WA, USA). The spleen was imaged with a linear transducer (L10-5) using pulse-inversion imaging at low mechanical index to minimize microbubble destruction¹⁷, starting 2 minutes after the beginning of the infusion. Each acquisition was repeated 3 times and the raw data were transferred to a PC for further quantification. Each cineloop was quantified using HDI Lab (Philips US, Bothell, WA, USA), a software that takes into account the compression map and allows quantification in linear units. The signal intensity was calculated from a region-of-interest (ROI) located in the subcapsular area. The ROI position was moved to compensate for the respiratory movements and subcutaneous artefacts. The time-intensity curve was exported into Deltagraph (Red Rock software, Salt Lake City, UT, USA). The data were fitted using a bi-exponential model. The quality of the fit was estimated using the correlation coefficient between the data and the model.

**Human spleen retrieval.** Spleens were retrieved and processed as described⁷. Medical and surgical care was not modified and patient written consent was obtained. The project was approved by the Ile-de-France II investigational review board. Upon a 30- to 90-minute period of warm ischemia linked to the surgical procedure, the main splenic artery was cannulated once the macroscopic aspect of the spleen had been examined by the pathologist, and a pre-experiment biopsy had been performed whenever required. The spleens were flushed with cold Krebs-albumin solution for transport to the laboratory.

**Parasites.** *Plasmodium falciparum* Palo-Alto (FUP-CB ¹³), D10 ¹⁸, and FCR-3 were cultured as described¹⁹. Panning on human amelanotic melanoma cells (C32)²⁰ were repeated until a cytoadherent rate of more than 5 iRBC per C32 cell was obtained. Cytoadherent iRBCs were amplified, grossly synchronized by gel flotation until complete reinvasion occurred over less than 16 hours, then stored in liquid nitrogen as large > 3 ml aliquots. Less than 7 days prior to the spleen challenge, aliquots were thawed, and parasites allowed to re-invade uninfected RBCs. Less than 14 hours prior to the spleen challenge, rings were eliminated by the gel flotation method, and mature forms allowed to re-invade in recently collected (< 7 days) and washed RBCs so as to obtain 7-40 ml of packed RBCs at 2 - 7% parasitaemia, that were washed once in RPMI prior to introduction into the perfusate.

***Ex-vivo* spleen perfusion.** Isolated-perfused spleen experiments were performed as described⁷, in a Plexiglas chamber maintained at 37°C by a regulated warmed air flow. Briefly, once in the laboratory (cold ischemia time, 60-90 minutes), the spleen was connected to the perfusion device, and a progressive warming from 6-10°C to 37°C was performed by increasing the Krebs-albumin medium flow from 1 ml/min to 50-150 ml/min over 40 to 60 minutes. During this adaptation period the patient's RBCs were flushed from the spleen (haematocrit at the end of the warming period < 0.1 %). When the spleen temperature reached 35°C, uninfected O+ RBCs were added (final hematocrit level of 5-10%) and allowed to circulate for 30 to 60 minutes. Glucose, Na and K concentrations, O₂ and CO₂ partial pressures, and pH were monitored in the vein, artery, and reservoir every 10-30 minutes using a iStat device (Abbott laboratories, Abbott Ppark, ILL, USA). At steady state, key physiologic markers were maintained in the following ranges: perfusate flow 0.8-1.2 ml/gr. of perfused spleen parenchyma/min, temperature of the spleen capsule 36.7-37.2°C, Na 135-148 mEq/l, K 4-5 mEq/L, Glucose 4-12 mmol/l, pH 7.2-7.35, haematocrit 4-10%, and arterial-venous oxygen partial pressure decay 60-120 mm Hg. Finally, a mixture of > 32-hour schizont-iRBCs (40±8 hrs post-invasion) and < 14-hour ring-iRBCs (7±7 hrs post-invasion) at 0.2 - 6% parasitaemia was introduced in the circulating system for 2 hours after normal RBCs had been rinsed out for 5-10 minutes (haematocrit prior to the introduction of iRBCs < 0.2%). Parasitaemia was quantified on Giemsa-stained thin smears or by flow cytometric analysis after staining of iRBC with Hoechst 33342 (diluted 1:1000; Molecular Probes, LSR, Becton-Dickinson, France). Data were analyzed using CELLQuest software (Becton-Dickinson, France).

**Parasite staging and counting.** The spleen tissue was fixed and processed exactly as described¹. The individual parasite nuclei, cytoplasm and malaria pigment were readily identified within the spleen tissue. iRBC stages were differentiated according to the following criteria. Ring-iRBC: light brown dot (nucleus) < 1µm with thin blue cytoplasm or pale round zone < ½ erythrocyte size. Schizont-iRBC: light brown dot > 1µm or > 1 brown dots with thick blue cytoplasm ≥ ½ erythrocyte size. Extra-erythrocytic parasite remnant: brown dot(s) not surrounded by red staining. For each spleen slide, iRBCs were counted within both the perifollicular zone and the red pulp adjacent to ≥ 3 follicles. Follicles with a clear differentiation between the white pulp, the adjacent perifollicular zone and the red pulp were selected for counting. Ring-and schizont-iRBC were counted and localized on ∼ 150 photographs (at least 4000 RBCs) for each spleen.

**Transmission and scanning electron microscopy.** At the end of perfusion, spleen samples (1mm³) were fixed overnight at 4°C in 2.5% glutaraldehyde and 1% paraformaldehyde in 0.08 M cacodylate buffer supplemented with CaCl2 (0.05%). For TEM, fixed samples were rinsed three times with 0.1 M sodium cacodylate buffer, postfixed with 1% osmium tetraoxide, 1.5% potassium ferricyanide in 0.1 M sodium cacodylate buffer for 1 hour at room temperature, and washed again with 0.1 M sodium cacodylate buffer. The samples were dehydrated through a graded series of ethanol bath (25% to 100%) and overnight in a mixture of Epon 812/propylene oxide at room temperature. After being embedded in Epon 812, samples were polymerized for 48 hours at 60°C. Ultrathin sections were prepared using a Leica ultracut UCT microtome and examined with a JEOL 1200 EX electron microscope operating at 80 kV. For SEM, fixed pieces were washed three times for 10 min in 0.1 M sodium cacodylate buffer, postfixed for 1 hour in 1% (w/v) osmium tetroxide, 1.5% potassium ferricyanide in 0.1 M sodium cacodylate buffer. Spleen sections were dehydrated through a graded series of 25%, 50%, 75% and 95% acetone solution for 10 minutes (each time). Samples were then dehydrated for 3x10 min in 100% acetone followed by critical point drying with CO2. Dried specimens were sputtered with 22nm gold palladium, examined and photographed with a JEOL JSM 6700F field emission scanning electron microscope operating at 5 kV or 7 kV. Images were acquired with the upper SE detector (SEI) and the lower secondary detector (LEI).

**Measurement of RBC deformability.** RBC and iRBC deformability were measured by ektacytometry using a laser-assisted optical rotational cell analyzer (LORCA®; Mechatronics, Hoorn, The Netherlands) as previously described ²¹. The unit of RBC deformability, namely the elongation index (E.I.) was defined as the ratio between the difference between the two axes of the ellipsoid diffraction pattern and the sum of these two axes. Red blood cell deformability was assessed over a range of shear stresses (0.3 to 30 Pascal) including 1.7 Pa, which corresponds approximately to the intravenous stress on the arterial side of the circulation 14, and 30 Pa, which occurs in the sinusoids of the spleen where RBCs have to squeeze through the small intercellular gaps.

**Erythrocyte surface immunofluorescence assay.** This assay was performed in order to detect surface-iRBCs parasite proteins, as described. iRBCs in suspension in PBS were prepared from cultures. Labeling of RBC surface parasite antigen was performed with sera from hyperimmune African adults (a kind gift from P. Druilhe; 1:100 serum dilution in PBS / 1% BSA) followed by Alexafluor 488-conjugated goat anti-human affinity-purified IgG (diluted 1:200; Molecular Probes, Eugene, OR). Parasite nuclei were stained with Hoechst 33342 (diluted 1:1000; Molecular Probes). Slides were mounted with Vectashield medium (Vector laboratories, Burlingame, CA). Images were acquired on a Zeiss Axiovert 200 M microscope, using an Axiocam HRc camera controlled by Zeiss Axiovision software (all from Carl Zeiss, Heidelberg, Germany).

**Surface iodination of iRBCs.** Highly synchronized mature stage iRBCs previously selected on cell C32 by panning procedure were enriched up to 80% by the gelatin flotation technique and then diluted with fresh erythrocytes (i.e., erythrocytes of less than 8 days or of 8 days of age, preferably erythrocytes of less than 8 days of age) to obtain approximately 20% ring-iRBCs at the next cycle. Surface iodination was done using the lactoperoxidase method²². The culture was stopped 14 h after reinvasion. Proteins were sequentially extracted with 1% Triton X-100, 2% SDS. Protease treatment of the samples (TPCK-treated trypsin and a-chymotrypsin TLCK (Sigma) was performed as described ²². lodinated samples were separated on a 5-17.5% gradient acrylamide gel. Autoradiography was done using Kodak Bio Max MS1 film. Pre-stained protein markers were purchased from Life Technologies (Gaithersburg, Maryland) and New England BioLabs Inc. (Beverly, Massachusetts).

**Cytoadherence assay.** The cytoadherence of iRBC to C32 cells, which express both the putative receptor molecule CD36 and intercellular adhesion molecule 1 (ICAM-1) was studied as previously described²⁰. In brief, a monolayer of C32 cells was prepared in 25 cm3 cell culture flask (Corning Incorporated, USA). RBC suspended at a concentration of 5 x 106 iRBC /ml in cytoadhesion medium at pH 6.8, were added to cell culture flask and gazed (1 % O₂, 3% CO₂, and 96% N₂) during 15 seconds, and this was incubated at 37°C for 15 min with gentle rocking every 5 minutes. At the end of the incubation, the cell culture flask was gently rinsed four times in RPMI 1640 medium. The monolayer was fixed in methanol, stained with 2% Giemsa stain, and examined microscopically. The number of iRBC adherent to 1000 melanoma cells was counted. Results were expressed as the number of iRBC which adhered to 100 C32 cells.

**Statistical analysis.** We used the student's paired t-test for statistical analysis; p values < 0.05 were considered significant. Compartment data analysis was performed using the WinNonLin software (version 5.1; Pharsight Corp., Mountain View, CA, USA).

### RESULTS

### Circulatory compartments in the human spleen

The circulatory pattern of the human spleen was explored using contrast-enhanced ultrasonography in 16 volunteers. Two minutes after starting the infusion of contrast agent (i.e, once a stable concentration of microbubbles was achieved), the spleen was studied using low mechanical index pulse inversion imaging. Despite the use of this low acoustic energy a certain amount of microbubbles was destroyed all along the continuous exposure of the spleen to the ultrasound beam. The time-intensity curve reflected the decrease in signal intensity (Figure 1, Material & Methods section). The model that displayed the best fit with the experimental decay in the 16 volunteers combined two exponentials (correlation coefficient R2 > 0.96 in all cases). The bi-exponential function can be expressed as N(t) = V₁(C₀exp(-β₁t)) + V₂(B + (C₀-B)exp(-β₂t)), where the first and second terms correspond to a slow, and a rapid flow compartment, respectively. C₀ refers to the initial (t=0) microbubble concentration, and was considered identical in both compartments. V₁ and V₂ refer to the relative fractional volumes (V₁ + V₂ = 1), and (β₁ and β₂ to the microbubble mean velocity in each compartment, respectively. Analysis of the experimental curves provided the following values: the Y intercept of each one-compartment exponential curve (Y1₀ and Y2₀) corresponds to CoV₁ and CoV₂, whereas 1/β₁ and 1/β₂ correspond to the exponential-characteristic times. In the slow compartment, the averaged (SD, range) relative fractional volume of the slow compartment was 70.6% (9.6, 51.5 - 84.4) and the averaged (SD, range) relative fractional flow was 10.12% (4.40, 3.99 - 16.47) (Figure 1). Altogether, these observations strongly support the existence of a dual microcirculatory organization of the human spleen (Figure 1), with approximately 10% of the blood input flowing through the slow compartment (Figures 1 &6).

### Clearance of iRBC by isolated-perfused human spleens

Isolated-perfused human spleens were perfused with highly synchronous parasite cultures at the schizont stage (40 ± 8 hrs post invasion) or at the ring stage (7 ± 7 hrs post invasion). Sequential Giemsa-stained thin films of the perfusate showed that circulating schizont-iRBC and -unexpectedly - ring-iRBCs parasitaemia rapidly decreased. Within 10 and 20 minutes, schizont-and ring-iRBC parasitaemias fell to 4.5% (range: 0-12.9) and 26.3% (range: 22.9-35.4%) of their initial values, respectively (Figure 2a). Complete clearance of the schizont-iRBCs was rapid, while ring-iRBC parasitaemia decreased at a slower rate and reached a plateau (Figure 2a). In contrast with schizont-iRBCs, ring-iRBCs displayed neither knobs nor iodinatable parasite proteins on their surface (Figure 8), and no cytoadherence was noticed on C32 human melanoma cells (data not shown), nor on spleen histological examination (Figure 4).

### Heterogeneity of ring-iRBCs revealed by the spleen challenge

Partial clearance of ring-iRBCs could reflect their slower circulation through the spleen or their stable retention in the spleen. To clarify this issue, ring-iRBCs preparations were split, with one part immediately perfused in the spleen and a second part kept at 37°C in Krebs-albumin medium (control "spleen naive" cells). Forty minutes (i.e. 40 spleen passages) after starting perfusion, the unretained cells were collected from the perfusate. These "spleen passaged" and the "spleen naive" populations were labeled with a different PKH each, pooled and reintroduced into the spleen. Sequential samples from the perfusion system were next analyzed using flow cytometry (Figure 3). The mean (SD) half-life of "spleen-naïve" ring-iRBCs (5.7 ± 3.1 minutes, two independent experiments) was similar to that observed during 6 previous experiments (see next section of the results "Modelling iRBC clearance"). In contrast, the "spleen passaged" ring-iRBCs were not cleared (Figure 3 b, c). This indicates that ring-iRBCs indeed consist of two distinct subpopulations, one retained in the spleen and one flowing through. This heterogeneity of the ring-iRBCs population with regard to spleen retention was stable over time in our culture conditions, and fairly independent of the initial parasitaemia introduced in the perfused spleen (Figure 2a). It was also independent of the parasite strain, since similar results were obtained with D10.

### Modelling iRBC clearance

The kinetics of iRBC parasitaemia in the perfusate was modeled using either a one- or two-compartment model, without or with a residual parasitaemia (plateau-phase) (Figure 3a1-3,). A one-compartment model without a residual parasitaemia had the best performance to describe schizont-iRBC kinetics (Figure 3a3). The kinetics of ring-iRBCs in the perfusate was best described by a one-order clearance phase of a "Retainable" subpopulation (∼ 74% of input) alongside a free circulation of a "Flow-Trough" subpopulation (∼ 26% of input) (Figure 3 and Table I). This model fitted with circulation-recirculation observations (see previous section) and moreover allowed a simple determination of the proportion of retained iRBCs. This is consistent with initial heterogeneity of the population of ring-iRBCs used for challenge evidenced above. The mean clearance half-life of the "Retainable" ring-iRBCs subpopulation (5.9 minutes, CI_{95%}: 2.0 -9.4), was ∼ 2-fold higher than the clearance half-life of schizont-iRBCs (2.8 minutes, CI_{95%}: 1.5 -3.9), suggesting at least partially different respective clearance mechanisms. The 5.9 minute clearance half-life of the retainable ring-iRBCs fits with the removal of ∼ 11 % of the retainable input at each spleen passage, assuming that each RBC crossed the isolated-perfused human spleen every minute⁷.

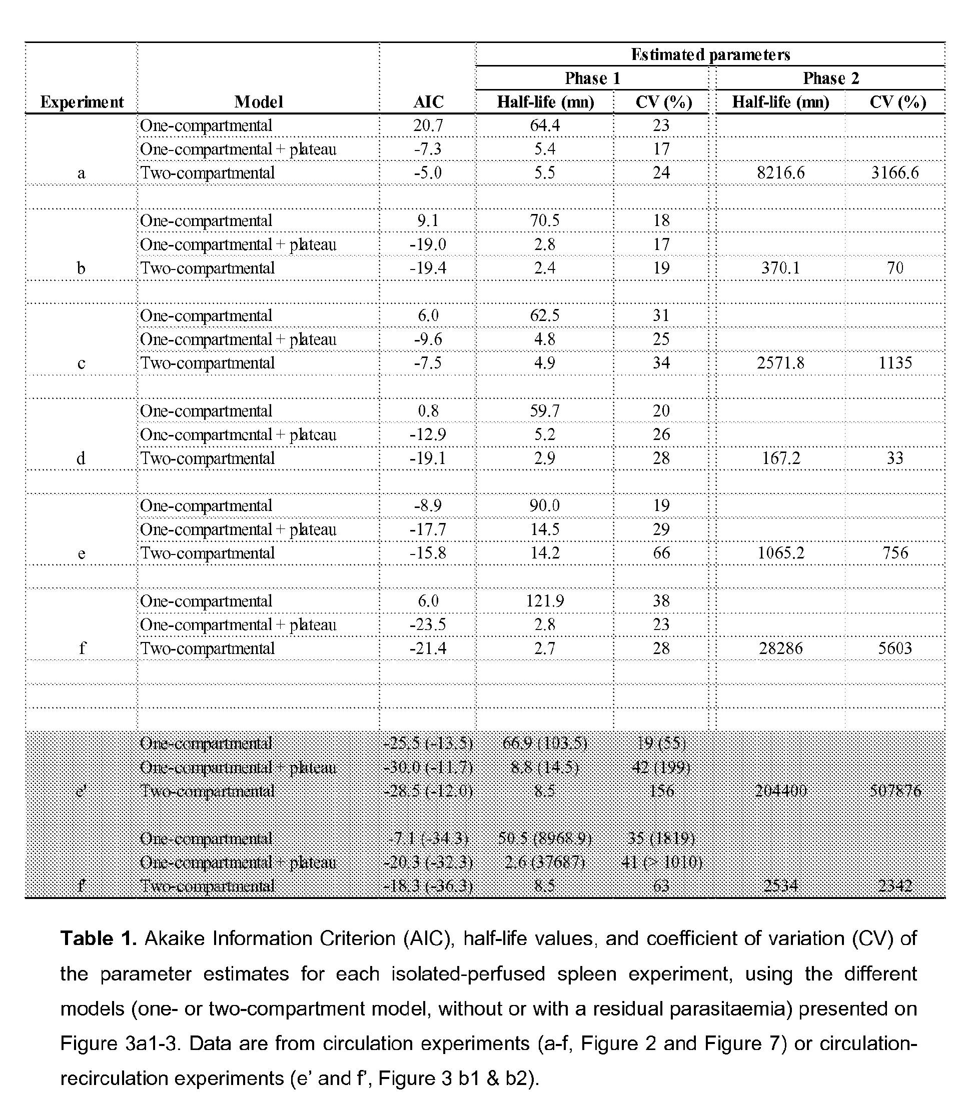

### Retention of ring-iRBCs in the human spleen

At the end of the experiments, the spleen tissue was processed for histological analysis and the distribution of ring- and schizont-iRBCs in the RP and perifollicular zone (PFZ) was quantified (Figure 4). The ring-iRBC tissue parasitaemia was significantly higher in the RP than in the PFZ both in Giemsa-stained (2.8% v. 1.1%, p = 0.00009, Figure 4a1-2) and Periodic Acid Schiff (PAS)-stained (3.0% vs.1.1%, p = 0.007, Figure 4b 1-2) sections. The mean (CI_{95%}) ring-iRBC retention index defined as the ratio ("tissue parasitaemia") /(circulating parasitaemia at the end of the experiment) was significantly higher in the RP (3.7; 1.9-5.4), than in the PFZ (1.4; 0.8-2.0, p = 0.002, Figure 4a3). The mean overall retention index of ring-iRBCs in the spleen (including both zones) was 2.5. Interestingly, the retention index of ring-iRBCs in the PFZ was close to 1, suggesting that - as previously observed with artesunate-exposed iRBCs ⁷ - the PFZ is more a transit zone than a retention/processing area for ring-iRBCs. The mean retention index of schizont-iRBCs was high (> 8) in both zones with a trend toward stronger retention in the RP. Use of PAS, which intensely stained the peculiar helix-shaped basal membrane of venous sinuses²³, allowed analysis of the distribution of ring-iRBCs in the sub-compartments of the RP, specifically, the cords, the sinus lumens and the abluminal side of the sinus walls (Figure 4c1). Most (86.0 ± 6.9%) ring-iRBCs were observed in the cords, whereas 14.0 ± 6.9% were in the sinus lumens. Significantly more ring-iRBCs were in direct contact with the PAS-stained basal membrane of venous sinuses (ie, immediately upstream of the narrow inter-endothelial slits), than in other sub-compartments of the RP (Figure 4c2). The very close proximity of knobless ring-iRBCs to the sinus basal membrane and inter-endothelial slits was confirmed by transmission electron microscopy (Figure 2b3 and Figure 4d1-2).

### Deformability of iRBCs

The elongation index (EI) of ring- or schizont-iRBCs populations was measured using LORCA. In order to infer the deformability at the level of a single iRBC, we measured the EI of the synchronized populations at different parasitaemia (3% to 80% for schizonts, 4% to 76% for rings, Figure 5A) and extrapolated to 100% parasitaemia. As expected, schizont-iRBCs had a very low elongation index (< 0.1 at 30 Pascal, 80% parasitaemia) across all shear stresses. At 30 Pascal (similar to that encountered in the splenic sinusoids ²⁴), ring-iRBCs were moderately but significantly less deformable than co-cultured co-perfused uninfected RBCs (Figure 4 A). When extrapolated to 100% iRBC population, the mean (CI_{95%}) EI of ring-iRBC was 0.47 (0.43-0.51), vs 0.58 for control uninfected RBCs (p< 0.0001 Figure 4 A3).

### DISCUSSION

The safe approaches used here provide novel, interconnected insights into the human spleen physiology and malaria pathophysiology, and raise a new paradigm whereby the ring stage population is heterogeneous, the less deformable subset being retained in the spleen. Contrast-enhanced ultras onography showed a two-compartment microcirculatory organization, with 10% of the blood input flowing through the slow compartment. A substantial proportion of ring-iRBCs was rapidly cleared by isolated-perfused human spleens. The 5.9 minute clearance half-life of the retainable ring-iRBC population fits with the removal of ∼ 10% of the input at each spleen passage, consistent with their retention in the slow circulatory compartment. In keeping with that interpretation, ring-iRBCs accumulated only in the RP, along the abluminal side of sinus walls. The deformability of ring-iRBCs was moderately but significantly reduced. Those rings were tiny, lacked knobs, did not cytoadhere in vitro and did not display any observable parasite-induced surface modification, excluding a PfEMP1-mediated retention mechanism. The overall picture indicates retention of ring-RBCs with reduced deformability upstream from the narrow inter-endothelial slits (i.e, the microcirculatory structures of the slow compartment that stringently challenge RBC deformability ^{11,25}) likely reflecting an original mechanism of micro-organism clearance, based on the new biophysical properties of the host cell.

A few exceptions apart ^{20,26}, ring-iRBCs have been considered an exclusively circulating component of the total body parasite biomass during human *Plasmodium falciparum* infection ²⁷. Their reduced deformability has been known for decades ^{15,28}, but deemed too mild to trigger spleen retention. However, experimental and clinical observations support the hypothesis that this phenomenon does occur during the course of acute human malaria. The ex-vivo spleen model filtered drug-exposed iRBCs at a rate similar to clearance rate in patients ⁷, and the efficient clearance of schizont-iRBCs observed here further validates this experimental set up. Importantly, a recent post-mortem study reported unexplained spleen retention of knobless iRBCs, with a mean sequestration index of 2.19 in the spleen (compared to 0.63 in the liver) ²⁹, a figure in line with the retention index of 2.5 determined here. We thus interpret these post-mortem observations as evidence of young ring-iRBC spleen retention in these patients. Another puzzling aspect of malaria pathogenesis can be revisited in light of our findings. The parasite biomass in *Plasmodium falciparum-infected* patients, as calculated from circulating ring-iRBCs was 2-fold lower than calculated from plasma levels of HRP-2, a parasite protein released by mature-iRBCs ²⁷. The relative deficit in parasite biomass estimated from the circulating ring stages may be accounted for by the pool of undetected ring-iRBCs retained in the spleen. Not least, retention in the spleen of the less deformable ring-iRBCs fits with available data linking reduced RBCs' mechanical deformability and spleen clearance in patients with RBC genetic disorders. Indeed, LORCA-based measurements in spleen-intact and splenectomized thalassemic patients has provided the only approximation of the EI below which RBC retention occurs in the spleen, namely 0.45 at 30 Pascal ³⁰. This is remarkably close to the value estimated here for ring-iRBCs (0.47 at 30 Pascal).

Because the LORCA measures the EI of a population of RBCs, the EI distribution (uni- or multi-modal) of individual ring-RBCs and the extent of individual variation is unknown. Deformability studies using single-cell tools have documented the reduced deformability of ring-iRBCs compared to uninfected RBCs from the same culture ^{15,19,28,31}, and furthermore evidenced wide variations between individual cells. Multiple parasite and/or host-cell factors could contribute to ring-iRBC heterogeneously reduced deformability. The erythrocyte population is known to be heterogeneous, circulating erythrocytes displaying different levels of maturation and senescence ³². As for the parasite, there is increasing evidence for heterogeneous expression by the individual merozoites of an array of surface molecules implicated in ligand-receptor interactions and of molecules discharged at the time of RBC merozoite invasion^{18,33}. Some of these interact with the erythrocyte cytoskeleton and contribute to host-cell membrane remodelling in young stages and to erythrocyte membrane stiffening after invasion^{13,19}. Others, such as Ring Surface Protein-2²⁰ predominantly associate with the surface of uninfected RBCs¹⁸, making their direct involvement in ring-iRBC spleen retention unlikely.

What is the ultimate fate of ring-iRBCs retained in the spleen RP: multiplication or destruction? A full intra-splenic maturation process of ring-iRBCs leading to intra-splenic reinvasion could theoretically take place, although the microenvironment in the cords of the RP has been considered hostile to RBCs³⁴. It is possible that spleen-retained ring-IRBCs are eventually phagocytosed as innate phagocytosis of ring-iRBCs has been observed during static in vitro experiments³⁵. Ring-iRBC destruction in the spleen is predicted to impact on the in vivo multiplication factor per cycle (IMF), which interestingly was lower (namely 3 - 16) in patients as assessed by counting circulating ring-iRBCs^{36,37} than predicted based on theoretical reinvasion rates (i.e. 18-24)³⁶. Such a discrepancy, usually interpreted as inefficient invasion in vivo, can also be viewed as resulting from spleen retention/clearance of a fraction of ring-iRBCs. Whatever the fate of ring-iRBCs within the spleen, their local retention reduces the parasite biomass that will sequester a few hours latter in vital organs such as the brain or the lungs.

Furthermore, cryptic ring-iRBCs retention in the spleen may explain why, in acute malaria, only a low proportion of the observed total RBC loss could be attributed to cumulated loss of iRBCs³⁸, since this was based on counting circulating ring-iRBC, ignoring the spleen-retained pool of ring-iRBCs.

Because they both implicate deformability-sensing mechanisms, spleen handling/clearance of ring-iRBCs and that of uninfected RBCs are linked. A link between spleen RBC filtration and parasite multiplication rate has been speculated^{15,39,40} but - in humans infected with *Plasmodium falciparum* - the iRBC population submitted to the spleen filtration process remained unidentified. Indeed, while mature-iRBCs are known to escape spleen filtration through sequestration, circulating ring-iRBCs visualized in the peripheral blood of patients were not considered susceptible to spleen retention. Our discovery that, shortly after invasion, a subpopulation of *Plasmodium falciparum* ring-iRBC is susceptible to spleen retention adds new strength to this link between RBC filtration and parasite multiplication. We thus infer that acquired or hereditary shift in the RBC deformability, as well as acquired or hereditary variation of the threshold for RBC retention by the spleen potentially impact on the clinical outcome of *Plasmodium falciparum* infection. Low-level spleen retention of ring-iRBCs may result in rapidly rising parasite loads leading to cerebral malaria or multiorgan failure, before anaemia and splenomegaly could develop. In contrast, high-level spleen retention of both RBCs and ring-iRBCs may generate a subacute infection accompanied by intra-splenic hemolysis, and favour the occurrence of severe anemia, frequently associated with a palpable splenomegaly. This could explain why severe anemia and cerebral malaria rarely occur simultaneously in the same patient. This novel concept triggers a shift in malaria research fields, such as modelling of infection kinetics, estimation of parasite loads, adjuvant therapy targeting RBC deformability⁴¹, analysis of risk factors for severe clinical forms⁴² (including patient age), or study of innate protection linked to hemoglobin inherited disorders.

### B. Example of a method for screening for compounds increasing rigidity of Plasmodium falciparum ring-infected red blood cells (ring-iRBCs)

**Parasite culture**. The Palo-Alto strain of *Plasmodium falciparum* (FUP)⁴⁴ is used. *Plasmodium falciparum* ring-iRBCs are cultured as previously described⁴⁵. Briefly, parasites are synchronised by selecting the ring stages by successive treatments (at least two successive treatments) with 5% (w/v) sorbitol (5 minutes at 37°C) in order to obtain their complete reinvasion in 4 hours. Ring-infected RBCs are then incubated at 37°C in complete medium (RPMI 1640 medium, bicarbonate, 25mM glutamine, 0.2% glucose, 100 µM hypoxanthine, 10 µg/ml gentamicine and 10% AB+ inactivated human serum pool) containing O+ RBCs in 75 cm² flasks treated for 30 seconds with an atmosphere of 1 % O₂, 3% CO₂ and 96% N₂.

***In vitro* test.** Schizonts (44 to 48H) are enriched by suspension in a gelatinous solution (Plasmion). They are then allowed to re-invade fresh O+ RBCs (i.e. O+ RBCs of less than 8 days or of 8 days of age, preferably O+ RBCs of less than 8 days of age) in complete medium at 37°C. Ten hours after the onset of incubation (age of 80-90% of the ring-stage RBCs), a compound to be tested is added to the culture, which is then further incubated for 2 hours at 37°C. After removal of the culture supernatant (by centrifugation at 1500 rounds per minute for 5 minutes), the pellet is resuspended in PBS (hematocrit level of 50%) and used to analyse deformability of ring-iRBCs.

### Analysis of Ring-iRBCs deformability

Deformability assessment is performed in several steps (see figure 9):
1. Use of a LORCA with a screening threshold for the decrease of the mean EI of 10%.
2. Analysis of the compounds identified by the LORCA technique as being able to increase rigidity of ring-iRBCs, by means of more accurate techniques, which enable to assess deformability of ring-iRBCs at an individual cellular scale (with a LORCA, analysis is performed at a populational cellular scale), e.g., by means of micropipets, optical tweezers, or a microfluidic device. These techniques allow to evaluate more precisely EI dispersion and thus the percentage of ring-iRBCs that could be retained in the spleen after exposure to a screened compound.
3. Optionally, few candidate compounds are tested using an isolated perfused human spleen model.

The LORCA technique is performed as previously described ⁴⁶. The principle of this method consists in submitting, to a shear force and to a constant temperature, a population of iRBCs and a population of uninfected RBCs, said populations being suspended in a viscous solution (5% polyvinylpyrrolidone in PBS, viscosity = 30mPa.sec at 37°C) and being introduced in a slit located between two concentric cylinders. The shear force resulting from the rotation of one of the cylinders (the outer one) leads to an elongation of the RBCs in the form of an ellipse. A laser beam emitted from a laser diode traverses the RBCs or iRBCs suspension and is diffracted by these cells in the volume. The laser beam diffraction pattern that is projected on a projection screen is captured by a digital video camera and transmitted to a computer. A software then converts the ellipsoid diffraction pattern into a digit value: the elongation index (EI). Thus, the deformability unit is defined as the ratio of height of the ellipse to [height + width of the ellipse] ^{46, 47}. This value is the mean IE value of the RBCs population (infected RBCs population or uninfected RBCs population) contained in the suspension. Deformability of infected RBCs and of uninfected RBCs is evaluated over a range of shear forces (0.3 to 30 Pascal), which include 1.7 Pa (shear force in the microvessels of the deep tissue) and 30 Pa (shear force in red pulp sinuses). Using one LORCA, one technician is able to test 30 compounds per days, i.e. at least 100 compounds per week, if one takes into account the steps of parasites preparation and storage of the generated data.

### REFERENCES

1. Maitland K, Marsh K. Pathophysiology of severe malaria in children. Acta Trop. 2004;90:131-140.
2. Wyler DJ, Gallin JI. Spleen-derived mononuclear cell chemotactic factor in malaria infections: a possible mechanism for splenic macrophage accumulation. J lmmunol. 1977;118:478-484.
3. David PH, Hommel M, Miller LH, Udeinya IJ, Oligino LD. Parasite sequestration in Plasmodium falciparum malaria: spleen and antibody modulation of cytoadherence of infected erythrocytes. Proc Natl Acad Sci U S A. 1983;80:5075-5079.
4. Boone KE, Watters DA. The incidence of malaria after splenectomy in Papua New Guinea. Bmj. 1995;311:1273.
5. Bach O, Baier M, Pullwitt A, et al. Falciparum malaria after splenectomy: a prospective controlled study of 33 previously splenectomized Malawian adults. Trans R Soc Trop Med Hyg. 2005;99:861-867.
6. Davidson RN, Wall RA. Prevention and management of infections in patients without a spleen. Clin Microbiol Infect. 2001;7:657-660.
7. Buffet PA, Milon G, Brousse V, et al. Ex vivo perfusion of human spleens maintains clearing and processing functions. Blood. 2006;107:3745-3752.
8. Looareesuwan S, Merry AH, Phillips RE, et al. Reduced erythrocyte survival following clearance of malarial parasitaemia in Thai patients. Br J Haematol. 1987;67:473-478.
9. Looareesuwan S, Davis TM, Pukrittayakamee S, et al. Erythrocyte survival in severe falciparum malaria. Acta Trop. 1991;48:263-270.
10. Urban BC, Hien TT, Day NP, et al. Fatal Plasmodium falciparum malaria causes specific patterns of splenic architectural disorganization. Infect lmmun. 2005;73:1986-1994.
11. Groom AC, Schmidt EE, MacDonald IC. Microcirculatory pathways and blood flow in spleen: new insights from washout kinetics, corrosion casts, and quantitative intravital videomicroscopy. Scanning Microsc. 1991;5:159-173; discussion 173-154.
12. Weiss L. Mechanisms of splenic control of murine malaria: cellular reactions of the spleen in lethal (strain 17XL) Plasmodium yoelii malaria in BALB/c mice, and the consequences of pre-infective splenectomy. Am J Trop Med Hyg. 1989;41:144-160.
13. Silva MD, Cooke BM, Guillotte M, et al. A role for the Plasmodium falciparum RESA protein in resistance against heat shock demonstrated using gene disruption. Mol Microbiol. 2005;56:990-1003.
14. Deitsch KW, Wellems TE. Membrane modifications in erythrocytes parasitized by Plasmodium falciparum. Mol Biochem Parasitol. 1996;76:1-10.
15. Cranston HA, Boylan CW, Carroll GL, et al. Plasmodium falciparum maturation abolishes physiologic red cell deformability. Science. 1984;223:400-403.
16. Chen LT. Microcirculation of the spleen: and open or closed circulation? Science. 1978;201:157-159.
17. Correas JM, Kurtisovski E, Bridal SL, Amararene A, Helenon O, Berger G. Optimizing an ultrasound contrast agent's stability using in vitro attenuation measurements. Invest Radiol. 2002;37:672-679.
18. Layez C, Nogueira P, Combes V, et al. Plasmodium falciparum rhoptry protein RSP2 triggers destruction of the erythroid lineage. Blood. 2005;106:3632-3638.
19. Mills JP, Diez-Silva M, Quinn DJ, et al. Effect of plasmodial RESA protein on deformability of human red blood cells harboring Plasmodium falciparum. Proc Natl Acad Sci U S A. 2007;104:9213-9217.
20. Pouvelle B, Buffet PA, Lepolard C, Scherf A, Gysin J. Cytoadhesion of Plasmodium falciparum ring-stage-infected erythrocytes. Nat Med. 2000;6:1264-1268.
21. Hardeman MR, Ince C. Clinical potential of in vitro measured red cell deformability, a myth? Clin Hemorheol Microcirc. 1999;21:277-284.
22. Buffet PA, Gamain B, Scheidig C, et al. Plasmodium falciparum domain mediating adhesion to chondroitin sulfate A: a receptor for human placental infection. Proc Natl Acad Sci USA. 1999;96:12743-12748.
23. Chen LT, Weiss L. Electron microscopy of the red pulp of human spleen. Am J Anat. 1972;134:425-457.
24. Dondorp AM, Kager PA, Vreeken J, White NJ. Abnormal blood flow and red blood cell deformability in severe malaria. Parasitol Today. 2000;16:228-232.
25. Weiss L, Chen LT. The differentiation of white pulp and red pulp in the spleen of human fetuses (72-145 mm crown-rump lenght). Am J Anat. 1974;141:393-413.
26. Silamut K, Phu NH, Whitty C, et al. A quantitative analysis of the microvascular sequestration of malaria parasites in the human brain. Am J Pathol. 1999;155:395-410.
27. Dondorp AM, Desakorn V, Pongtavornpinyo W, et al. Estimation of the total parasite biomass in acute falciparum malaria from plasma PfHRP2. PLoS Med. 2005;2:e204.
28. Nash GB, O'Brien E, Gordon-Smith EC, Dormandy JA. Abnormalities in the mechanical properties of red blood cells caused by Plasmodium falciparum. Blood. 1989;74:855-861.
29. Prommano O, Chaisri U, Turner GD, et al. A quantitative ultrastructural study of the liver and the spleen in fatal falciparum malaria. Southeast Asian J Trop Med Public Health. 2005;36:1359-1370.
30. Dondorp AM, Chotivanich KT, Fucharoen S, et al. Red cell deformability, splenic function and anemia in thalassaemia. Br J Haematol. 1999;105:505-508.
31. Li J, Dao M, Lim CT, Suresh S. Spectrin-Level Modeling of the Cytoskeleton and Optical Tweezers Stretching of the Erythrocyte. Biophys J. 2005.
32. Lutz HU. Innate immune and non-immune mediators of erythrocyte clearance. Cell Mol Biol (Noisy-le-grand). 2004;50:107-116.
33. Cortes A, Benet A, Cooke BM, Barnwell JW, Reeder JC. Ability of Plasmodium falciparum to invade Southeast Asian ovalocytes varies between parasite lines. Blood. 2004;104:2961-2966.
34. Levesque MJ, Groom AC. pH environmental of red cells in the spleen. Am J Physiol. 1976;231:1672-1678.
35. Ayi K, Turrini F, Piga A, Arese P. Enhanced phagocytosis of ring-parasitized mutant erythrocytes: a common mechanism that may explain protection against falciparum malaria in sickle trait and beta-thalassemia trait. Blood. 2004;104:3364-3371.
36. White NJ, Chapman D, Watt G. The effects of multiplication and synchronicity on the vascular distribution of parasites in falciparum malaria. Trans R Soc Trop Med Hyg. 1992;86:590-597.
37. Simpson JA, Aarons L, Collins WE, Jeffery GM, White NJ. Population dynamics of untreated Plasmodium falciparum malaria within the adult human host during the expansion phase of the infection. Parasitology. 2002;124:247-263.
38. Price RN, Simpson JA, Nosten F, et al. Factors contributing to anemia after uncomplicated falciparum malaria. Am J Trop Med Hyg. 2001;65:614-622.
39. Looareesuwan S, Ho M, Wattanagoon Y, et al. Dynamic alteration in splenic function during acute falciparum malaria. N Engl J Med. 1987;317:675-679.
40. Wyler DJ. The spleen in malaria. Ciba Found Symp. 1983;94:98-116.
41. Dondorp AM, Omodeo-Sale F, Chotivanich K, Taramelli D, White NJ. Oxidative stress and rheology in severe malaria. Redox Rep. 2003;8:292-294. 22
42. Schellenberg D, Menendez C, Kahigwa E, et al. African children with malaria in an area of intense Plasmodium falciparum transmission: features on admission to the hospital and risk factors for death. Am J Trop Med Hyg. 1999;61:431-438.
43. Arditi M, Frinking PJ, Zhou X, Rognin NG. A new formalism for the quantification of tissue perfusion by the destruction-replenishment method in contrast ultrasound imaging. IEEE Trans Ultrason Ferroelectr Freq Control. 2006;53:1118-1129.
44. Gysin J, Fandeur T. (1983). Saimiri sciureus (karyotype 14-7): an alternative experimental model of Plasmodium falciparum infection. Am J Trop Med Hyg. 32(3):461-7.
45. Gay F, Robert C, Pouvelle B, Peyrol S, Scherf A, Gysin J. (1995). Isolation and characterization of brain microvascular endothelial cells from Saimiri monkeys. An in vitro model for sequestration of Plasmodium falciparum-infected erythrocytes: J lmmunol Methods. 184(1):15-28.
46. Hardeman MR, Goedhart PT, Dobbe JGG, Lettinga KP. (1994). Laser-assisted optical rotational cell analyser (LORCA). 1. A new instrument for measurement of various structural hemorheologic parameters. Clin Hemorheol. 14: 605-618.
47. Hardeman MR, Goedhart PT, Schut. NH. (1994). Laser assisted Optical Rotational Cell Analyser. (L.O.R.C.A.) Red blood cell deformability: Elongation index versus cell transit time. Clin Hemorheol. 14(4) : 619-630.

## Claims

1. A method for screening compounds for their ability to increase rigidity of red blood cells (RBC) infected by a protozoan parasite of the genus *Plasmodium*, said method comprising or consisting of the following steps:
a) culturing RBCs infected by said parasite and optionally and separately culturing uninfected RBCs, each culture being carried out both in the presence and in the absence of a compound to be tested for its ability to increase rigidity of infected RBCs (iRBCs) and;
b) measuring the deformability of one or several iRBCs cultured in the presence of said compound, and one or several iRBCs cultured in the absence of said compound; and,
c) optionally, measuring the deformability of one or several uninfected RBCs cultured in the presence of said compound, and one or several uninfected RBCs cultured in the absence of said compound,
wherein a decrease by at least 5%, preferably at least 10% and more preferably at least 15%, of the deformability of iRBCs cultured in the presence of a compound in comparison with the deformability of iRBCs cultured in the absence of the same compound is indicative that said compound is able to increase rigidity of iRBCs.

2. The method according to claim 1, wherein said iRBCs are at the ring stage.

3. The method according to claim 1 or 2, wherein the iRBCs used at step a) where obtained by the following step:
- infecting RBCs, preferably RBCs of less than 8 days of age, with iRBCs, in particular with a culture of iRBCs in which the schizont-stage iRBCs have been enriched, said enrichment being performed for example by plasmion treatment.

4. The method according to any one of claims 1 to 3, wherein step a) and/or the step of claim 3 is (are) preceded by the following step:
- synchronization of the parasites of the iRBCs or of the parasites of the schizont-enriched iRBCs culture, for example by one or several sorbitol treatment(s).

5. The method according to any one of claims 1 to 4, wherein
- one screens the deformability of uninfected RBCs cultured in the presence of the compound which is approximately the same or differs by less than 5% from that of the uninfected RBCs cultured in the absence of the compound, and
- optionally, one screens the deformability of schizont-iRBCs cultured in the presence of the compound which is approximately the same or differs by less than 5% from that of the uninfected schizont-iRBCs cultured in the absence of the compound.

6. The method according to any one of claims 1 to 5, wherein the screening performed is a low throughput screening or a high throughput screening.

7. The method according to any one of claims 1 to 6, wherein the deformability of RBCs and iRBCs is measured on a populational cellular scale and/or on an individual cellular scale.

8. The method according to any one of claims 1 to 7, wherein the deformability of RBCs and iRBCs is measured by means of a rheoscope, an ektacytometer, a microfluidic device, a micropipet and/or an optical tweezer

9. The method according to claim 8, wherein the ektacytometer is a commercial automated instrument, preferably a Laser-assisted Optical Rotational Cell Analyzer (LORCA; Mechatronics, Hoorn, Netherlands) or a RHEODYN-SSD (Myrenne, Roetgen, Germany), and more preferably a LORCA.

10. The method according to claim 8 or 9, wherein deformability of RBCs and iRBCs is measured over a range of shear stresses from 0.3 to 30 Pascal (Pa), including 1.7 Pa, and 30 Pa.

11. The method according to any one of claims 1 to 10, wherein the parasitemia level of iRBCs is of at least 20% or 30% of the cell population, preferably at least 50% and more preferably at least 70% of the cell population.

12. The method according to any one of claims 1 to 11, wherein the measure of deformability is carried out by reference to the elongation index (EI).

13. The method according to claim 12, wherein an EI below 0.43, more preferably below 0.42, for iRBCs cultured in the presence of the compound, when extrapolated at 100% parasitemia, at 30 Pa, is indicative that said compound is able to increase rigidity of iRBC.

14. The method according to any one of claims 1 to 13, wherein said uninfected and infected RBCs are human RBCs.

15. The method according to any one of claims 1 to 14, wherein said parasite is chosen in the group consisting of *Plasmodium falciparum, Plasmodium vivax, Plasmodium ovale, Plasmodium malariae, Plasmodium knowlesi, Plasmodium inui, Plasmodium cynomolgi, Plasmodium simiovale, Plasmodium brazilianum, Plasmodium schwetzi* and *Plasmodium simium*, preferably in the group consisting of *Plasmodium falciparum, Plasmodium vivax, Plasmodium ovale, Plasmodium knowlesi*, and *Plasmodium malariae*, and more preferably in the group consisting of *Plasmodium falciparum* and *Plasmodium vivax*.

16. The method according to any one of claims 1 to 15, wherein said parasite is *Plasmodium falciparum*, in particular the Palo Alto I strain of *Plasmodium falciparum*.

17. The method according to any one of claims 1 to 16, further comprising a step of validating a screened compound with the isolated perfused human spleen model and/or an isolated perfused pig spleen model.

18. The method according to any one of claims 1 to 17, wherein the screened compounds are capable of interacting with the iRBCs membrane its cortical cytoskeleton included and/or entering into the iRBCs, in particular crossing the iRBCs lipid bilayer, or interact with the modified bilayer itself.

19. The application of the method according to any one of claims 1 to 18, for the selection of compounds which selectively interact with red blood cells (RBCs) infected by a protozoan parasite of the genus *Plasmodium* (iRBCs) or selectively interact with iRBCs at the ring stage, and are suitable to increase their rigidity.
